(19) Europäisches Patentamt
European Patent Office
Office européen des brevets

(11) Veröffentlichungsnummer: **0 174 272**
**B1**

(12) # EUROPÄISCHE PATENTSCHRIFT

(45) Veröffentlichungstag der Patentschrift: **31.08.88**

(51) Int. Cl.⁴: **C 07 C 51/56**, C 07 C 51/567, C 07 C 57/13

(21) Anmeldenummer: **85810366.6**

(22) Anmeldetag: **09.08.85**

(54) **Verfahren zur Herstellung von Dimethylmaleinsäureanhydrid.**

(30) Priorität: **15.08.84 CH 3909/84**

(43) Veröffentlichungstag der Anmeldung: **12.03.86 Patentblatt 86/11**

(45) Bekanntmachung des Hinweises auf die Patenterteilung: **31.08.88 Patentblatt 88/35**

(84) Benannte Vertragsstaaten: **BE CH DE FR GB IT LI NL**

(56) Entgegenhaltungen:
**EP-A-0 078 239**
**GB-A-870 681**

**HELVETICA CHIMICA ACTA, Band 61, Nr. 7, November 1978, Seiten 2751-2753; M.E. BAUMANN et al.: "Eine decatboxylative Dimerisierung von Maleinsäureanhydrid zu Dimethylmaleinsäueanhydrid"**

(73) Patentinhaber: **CIBA- GEIGY AG, Klybeckstrasse 141, CH- 4002 Basel (CH)**

(72) Erfinder: **Baumann, Marcus, Dr., Hammerstrasse 82, CH- 4057 Basel (CH)**
Erfinder: **Breitenstein, Werner, Dr., St. Galler Ring 179, CH- 4054 Basel (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 174 272 B1**

LIBER, STOCKHOLM 1988

**Beschreibung**

Die vorliegende Erfindung betrifft ein verfahren zur Herstellung von Dimethylmaleinsäureanhydrid durch Umsetzung von Maleinsäure, Fumarsäure, Maleinsäureanhydrid oder Gemischen hiervon in Gegenwart von N-acylierten heterocyclischen Amidinen oder deren Salze bei erhöhten Temperaturen.

Aus der DE-A-2 233 862 und DE-A-2 233 889 sind Verfahren zur Herstellung von Dimethylmaleinsäureanhydrid aus 2 Mol Maleinsäureanhydrid, Maleinsäure und/der Fumarsäure bekannt, wobei die Umsetzung bei erhöhten Temperaturen in Gegenwart von mindestens 1 Mol Amidin mit einem primären oder sekundären N-Atom durchgeführt wird. Gute Ausbeuten werden nur dann erzielt, wenn das Reaktionsgemisch einer sauren Hydrolyse unterzogen wird. Ferner wird die verwendete hohe Menge an Amidin als nachteilig empfunden.

Gegenstand vorliegender Erfindung ist ein Verfahren zur Herstellung von Dimetylmaleinsäureanhydrid aus 2 Äquivalenten Maleinsäure, Fumarsäure und/oder Maleinsäureanhydrid in Gegenwart eines Amidines, eines Amidinsalzes oder Gemisches davon und bei Temperatüren von mindestens 90° C, das dadurch gekennzeichnet ist, dass das Amidin bzw. das Amidinsalz den Formeln I bzw. II

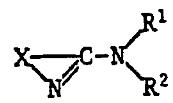

(I)

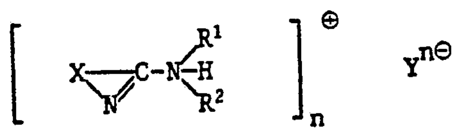

(II)

entspricht, worin

$R^1$ Acyl und

$R^2$ ein Wasserstoffatom, $C_1$-$C_{12}$-Alkyl, $C_5$-$C_7$-Cycloalkyl, $C_6$-$C_{12}$-Aryl, $C_7$-$C_{16}$-Aralkyl, $C_8$-$C_{16}$-Alkaralkyl oder Acyl oder

$R^1$ und $R^2$ zusammen der Diacylrest einer 1,2-Dicarbonsäure sind,

Y das Anion einer anorganischen oder organischen Protonensäure und

n eine Zahl von 1 bis 3 bedeuten, und X Zusammen mit der Gruppierung

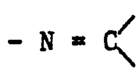

den Rest eines substituierten oder unsubstituierten 5- oder 6-gliedrigen Heteroringes bildet, der weitere Heteroatome enthalten kann.

Vorzugsweise verwendet man im erfindungsgemässen Verfahren Maleinsäure, Maleinsäureanhydrid oder 1:1-Gemische hiervon (Molverhältnis).

Als durch X zusammen mit der Gruppierung

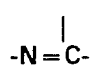

gebildete Reste eines 5

oder 6-gliedrigen, gegebenenfalls weitersubstituierten Heteroringes, der weitere Heteroatome enthalten kann, sind beispielsweise Imidazolyl-, Pyrazolyl-, Triazolyl-, Thiazolyl-, Isothiazolyl-, Oxadiazolyl-, Thiadiazolyl-, Oxazolyl-, Pyridinyl-, Pyridazinyl-, Pyrimidinyl-, Pyrazinyl- und Triazinylreste zu nennen.

Sind diese Reste weitersubstituiert, so können sie beispielsweise Halogene, wie Fluor, Chlor oder Brom, Phenylgruppen, Alkyl- oder Alkoxygruppen mit 1 - 4 Kohlenstoffatomen, Aminogruppen, Mono- oder Dialkylaminogruppen mit je 1 - 4 Kohlenstoffatomen im Alkylrest oder Hydroxylgruppen enthalten, oder aber mit weiteren homo- oder heterocyclischen Ringen kondensiert sein. Bevorzugte Substituenten sind Halogen, $C_1$-$C_4$-Alkyl und $C_1$-$C_4$-Alkoxy. Als Beispiele kondensierter 5- oder 6-gliedriger Heteroringsysteme seien genannt: Benzimidazol, Benzthiazol, Benzoxazol, Pterin, Purin, Chinolin, Isochinolin, Naphthyridin, Phthalazin, Cinnolin, Chinazolin und Chinoxalin.

Vorzugsweise sind durch X zusammen mit der Gruppierung

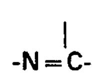

dargestellte Reste eines 5- oder 6-gliedrigen Heteroringes nicht weitersubstituiert. Bevorzugt ist der Heteroring ein Thiazolyl-2-Rest, besonders der Pyridinyl-2-Rest.

Die Amidine der Formel I sind bekannt oder können auf an sich bekannte Weise durch Acylierung von Verbindungen der Formel Ia

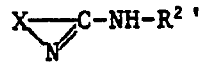

(Ia),

worin X die unter Formel I angegebene Bedeutung hat und $R^{2'}$ ein Wasserstoffatom oder einen $R^2$ entsprechenden Rest bedeutet, hergestellt werden. Als Beispiele geeigneter Ausgangs-Amidine der Formel Ia seien genannt: 2-Aminoimidazol, 2-Aminobenzimidazol, 3-Aminopyrazol, 3-Amino-5-methylpyrazol, 3-Amino-4-brom-5-methylpyrazol, 3-Amino-1-phenylpyrazol, 3-Amino-1,2,4-triazol, 3,5-Diamino-1,2,4-triazol, 4-Amino-1,2,3-triazol, 2-Amino-1,3-thiazol, 3-Aminoisothiazol, 2-Amino-5-chlorthiazol, 2-Amino-4-phenylthiazol, 2-Aminobenzthiazol, 2-Amino-6-brombenzthiazol, 2-Amino-4,6-dibrombenzthiazol, 3-Amino-4-phenylfurazan, 3-Amino-4-methylfurazan, 3-Aminoisoxazol, 2-Aminooxazol, 2-Aminobenzoxazol, 2-Aminopyridin, 2-Amino-3-methyl-, -4-methyl-oder -6-methylpyridin, 2-Amino-5-brompyridin, 2-Amino-6-brompyridin, 2-Amino-5-chlorpyridin, 2-Amino-3,5-dibrompyridin, 2-Amino-3,5-dichlorpyridin, 2-Amino-3-methylaminopyridin, 2,6-Diaminopyridin, 2,3-Diaminopyridin, 2-Aminopyrazin, 2-Aminopyrimidin, 6-Amino-2-chlorpyrimidin, 6-Amino-2,4-dimethylpyrimidin, 2-Amino-5-brom-4,6-dimethylpyrimidin, 2-Amino-6-chlorpyrimidin, 2-Amino-4,6-dichlorpyrimidin, 6-Amino-2,4-dichlorpyrimidin, 2-Amino-4,6-dimethylpyrimidin, 4,6-Diaminopyrimidin,6-Amino-4-methylpyrimidin, 3-Aminopyridazin, 2-Amino-1,3,5-triazin, 2,4,6-Triamino-1,3,5-triazin, 2-Amino-4,6-dichlor-1,3,5-triazin, 2-Amino-4,6-dimethyl-1,3,5-triazin, 4-Amino-6-hydroxy-2-methyl-1,3,5-triazin, 2,4-Diamino-6-methyl-1,3,5-triazin, 8-Aminopurin, 2-Aminopurin, 6-Aminopurin (Adenin), 2-Amino-6-brompurin, 2-Amino-6-chlorpurin, 6-Amino-2,8-dichlorpurin, 8-Amino-2,6-dichlorpurin, 6-Amino-2-methylpurin, 2,8-Diaminopurin, 6,8-Diaminopurin, 7-Methyl-2,6,8-triaminopurin, 1-Aminoisochinolin, 2-Aminochinolin, 2,4-Diaminochinolin, 2-Amino-1,7-naphthyridin, 2-Amino-1,5-naphthyridin, 2-Amino-6,7-dimethyl-1,8-naphthyridin, 2-Aminochinoxalin, 2,3-Diaminochinoxalin, 4-Aminochinazolin.

Werden im erfindungsgemässen Verfahren Amidinsalze der Formel II verwendet, so stellt n eine ganze Zahl von 1 bis 3 und Y vorzugsweise das Anion der Ameisen-, Essig-, Propion-, Chlorwasserstoff-, Bromwasserstoff-, Schwefel- oder Phosphorsäure dar. Ganz besonders bevorzugt ist Y jedoch das Anion einer aliphatischen Monocarbonsäure mit 2 bis 4 C-Atomen, besonders der Essigsäure ($n=1$). Diese Salze können in üblicher Weise durch Behandeln des betreffenden Amidins der Formel I mit der entsprechenden Säure hergestellt werden. Diese Herstellung kann direkt in situ erfolgen, oder man kann das isolierte Salz für die Umsetzung verwenden.

Wenn $R^2$ $C_1$-$C_{12}$-Alkyl ist, kann dieser Rest verzweigt oder besonders linear sein, vorzugsweise $C_1$-$C_6$-Alkyl. Beispiele geeigneter Reste $R^2$ sind Methyl, Ethyl, n-Propyl, i-Propyl, n-Butyl, i-Butyl, t-Butyl, Pentyl, Hexyl, Octyl, Decyl, Dodecyl, Cyclopentyl, Cyclohexyl, Cycloheptyl, Phenyl, Naphthyl, Benzyl, 2-Phenylethyl, Methylphenyl, Ethylphenyl und Methylbenzyl. Vorzugsweise ist $R^2$ ein Wasserstoffatom.

$R^1$ und $R^2$ als Acyl enthalten bevorzugt 1 bis 12 C-Atome. Das Acyl kann der Formel $R^3CO$ entsprechen, worin $R^3$ lineares oder verzweigtes Alkyl mit vorzugsweise 1 bis 6 C-Atomen, Cycloalkyl mit vorzugsweise 5 bis 7 Ringkohlenstoffatomen, Aryl mit 6-12 C-Atomen oder Aralkyl mit 7 bis 12 C-Atomen ist. Beispiele für Acyl sind Formyl, Acetyl, Propionyl und Benzoyl, besonders bevorzugt sind Acetyl und Propionyl.

Wenn $R^1$ und $R^2$ zusammen der Diacylrest einer 1,2-Dicarbonsäure sind, bildet der Rest mit dem N-Atom, an das $R^1$ und $R^2$ gebunden sind, einen 5-gliedrigen Imidring. Der Diacylrest kann z. B. durch $C_1$-$C_{12}$-Alkyl, Halogen, besonders Chlor, Cyano oder Phenyl substituiert sein und der Formel -CO-$R^4$-CO- entsprechen, worin $R^4$ ein zweiwertiger aliphatischer oder aromatischer Kohlenwasserstoffrest ist, an den die beiden Carbonylgruppen in 1,2-Stellung gebunden sind. Geeignete derartige Reste sind z. B. 1,2-Cyclohexylen, 1-2-Cyclopentylen, Ethylen, Ethenylen und 1,2-Phenylen. 1,2-Dicarbonsäuren, von denen sich der Diacylrest ableitet, sind z. B. 1,2-Cyclopentan- und 1-2-Cyclohexandicarbonsäure, Bernsteinsäure, alkylierte Bernsteinsäuren, wie z. B. Methyl-, Dimethyl-, Ethyl-, Propyl-, i-Propyl-, Butyl-, Pentyl-, Hexyl-, Octyl-, Decyl-, Dodecyl-, Phenyl- oder Diphenylbernsteinsäure, Maleinsäuren, wie z. B. Maleinsäure, Methyl-, Dimethyl-, Ethyl-, Propyl, Phenyl-, Diphenyl-, Cyano-, 1-Phenyl-2-methyl-, 1-Methyl-2-cyanomaleinsäure, Phthalsäure, Chlorphthalsäure, Dichlorphthalsäure, Tetrachlor- oder Tetrabromphthalsäure. Besonders bevorzugt sind $R^1$ und $R^2$ zusammen als Diacylrest von Phthalsäuren, Maleinsäuren oder Bernsteinsäuren abgeleitet.

Die erfindungsgemässe Umsetzung kann in einem gegenüber den Reaktionspartnern inerten organischen Lösungsmittel, wie gegebenenfalls chlorierten aromatischen Kohlenwasserstoffen, z. B. Benzol, Toluol, Xylolen, Chlorbenzol oder Dichlorbenzolen, Dialkylsulfoxiden, z. B. Dimethylsulfoxid, Methylcellosolve, Hexamethylphosphorsäuretriamid, N,N-Dislkylamiden einer niederen Monocarbonsäure, z. B. Dimethylformamid oder Dimethylacetamid, oder niederen Dialkylestern der Kohlensäure, z. B. Dimethylcarbonat oder Diethylcarbonat, durchgeführt werden. Auch Gemische solcher Lösungsmittel können verwendet werden. Falls das Amidinsalz der allgemeinen Formel II direkt in situ hergestellt wird, kann gegebenenfalls auch die verwendete Säure, z. B. eine aliphatische $C_2$-$C_4$-Carbonsäure, besonders Essigsäure,

als Lösungsmittel verwendet werden.

Gemäss einer bevorzugten Ausführungsform wird die erfindungsgemässe Umsetzung ohne Zusatz eines Lösungsmittels oder insbesondere in wasserfreier Essigsäure durchgeführt.

Die Reaktionstemperatur beträgt vorzugsweise 90 bis 200°C, besonders 110 bis 180°C. Die Reaktion kann gegebenenfalls unter Druck durchgeführt werden.

Dem Reaktionsgemisch kann auch eine Pufferverbindung, z. B. ein Alkaliacetat, wie Natriumacetat, zugesetzt werden. Bei alleiniger Verwendung von Maleinsäureanhydrid hat es sich als zweckmässig erwiesen, Wasser mitzuverwenden, vorteilhaft in einer Menge von 0,5 bis 20 Gew.-%, bezogen auf die Menge des eingesetzten Maleinsäureanhydrides.

Die Verbindungen der Formeln I und II können in Mengen bis zu 1 Mol pro 2 Mol Maleinsäure, Fumarsäure und/oder Maleinsäureanhydrid eingesetzt werden. Überraschend wurde gefunden, dass auch die Verwendung katalytischer Mengen Amidin bzw. Amidinsalz der Formeln I bzw. II genügt und die Reaktion auch beim Einsatz von vorzugsweise 1 bis 20 Mol-%, besonders 1 bis 10 Mol-% und ganz besonders 5 bis 10 Mol-%, bezogen auf die Reaktanden, zu hohen Ausbeuten führt. Die Verwendung katalytischer Mengen ist daher bevorzugt.

Die Isolierung und Reinigung des Reaktionsproduktes erfolgen nach üblichen Methoden, z. B. Destillation, Wasserdampfdestillation, Extraktion oder Kristallisation. Es ist ein besonderer Vorteil des erfindungsgemässen Verfahrens, dass die Isolierung des Reaktionsproduktes direkt vorgenommen werden kann, ohne dass eine saure Hydrolyse durchgeführt werden muss und hierbei hohe Ausbeuten erzielt werden können. Die Amidinverbindungen können hierbei quantitativ zurückgewonnen werden.

Dimethylmaleinsäureanhydrid ist ein wertvolles Zwischenprodukt zur Herstellung von lichtempfindlichen Polymeren mit Dimethylmaleinimidylgruppen (vgl. DE-A-2 626 769).

Die nachfolgenden Beispiele erläutern die Erfindung näher. Die Prozentangaben sind Gewichtsprozent.

**Beispiel 1:**

116 g (1,0 Mol) Maleinsäure und 20,2 g (0,1 Mol) N-(Pyrid-2-yl)-3,4-dimethylmaleinimid werden in 300 ml Eisessig 48 Stunden am Rückfluss gekocht. Anschliessend wird der Rückstand wasserdampfdestilliert. Aus dem Destillat werden nach Filtration und Trocknen 42,8 g (48 %) Dimethylmaleinsäureanhydrid vom Schmelzpunkt (Smp.) 91-93°C erhalten. Durch Extraktion der verbliebenen Wasserphase mit Ether werden weitere 7,89 g (12,3 %) Dimethylmaleinsäureanhydrid isoliert.

**Beispiel 2:**

Zu einer kochenden Lösung von 20,2 g (0,1 Mol) N-(Pyrid-2-yl)-3,4-dimethylmaleinimid und 9 ml (0,5 Mol) Wasser in 400 ml Eisessig werden innert einer Stunde 98,0 g (1,0 Mol) Maleinsäureanhydrid in Portionen gegeben. Danach wird 22 Stunden am Rückfluss gekocht. Nach Aufarbeiten wie in Beispiel 1 angegeben werden 52,4 g (63 %) Dimethylmaleinsäureanhydrid vom Smp. 92-93°C erhalten.

**Beispiel 3:**

Zu einer kochenden Lösung von 20,2 g (0,1 Mol) N-(Pyrid-2-yl)-3,4-dimethylmaleinimid in 100 ml Eisessig wird innert 20 Minuten eine Lösung von 98 g (1,0 Mol) Maleinsäureanhydrid in 300 ml Eisessig getropft. Anschliessend wird innert 1,5 Stunden eine Lösung von 9 ml (0,5 Mol) Wasser in 50 ml Eisessig zugetropft und 20 Stunden weiter gekocht. Nach Aufarbeiten wie in Beispiel 1 angegeben, werden 50,8 g (60,6 %) Dimethylmaleinsäureanhydrid vom Smp. 91-93°C erhalten.

**Beispiel 4:**

Eine Lösung von 58,0 g (0,5 Mol) Maleinsäure, 49,0 g (0,5 Mol) Maleinsäureanhydrid und 20,2 g (0,1 Mol) N-(Pyrid-2-yl)-3,4-dimethylmaleinimid in 300 ml Eisessig wird 6 Stunden am Rückfluss gekocht. Nach Entfernen des Eisessigs wird der Rückstand 15 Minuten auf 150°C erwärmt. Anschliessend wird wasserdampfdestilliert und das gebildete Dimethylmaleinsäureanhydrid wie in Beispiel 1 angegeben isoliert. Ausbeute: 37,1 g (39 %).

**Beispiele 5-14:**

Eine Lösung von 58 g (0,5 Mol) Maleinsäure, 49 g (0,5 Mol) Maleinsäureanhydrid und das Amidin bzw. Amidinsalz werden in 300 ml Eisessig gelöst und 48 Stunden am Rückfluss erhitzt. Nach Entfernen des Eisessigs wird der Rückstand wasserdampfdestilliert und das gebildete Dimethylmaleinsäureanhydrid (DMA) gemäss Beispiel 1 isoliert. Weitere Angaben finden sich in nachfolgender Tabelle.

| Beispiel | Amidin bzw. Amidinsalz | Menge (Mol-%) | Ausbeute DMA (Gewichts-prozent) |
|---|---|---|---|
| 5 | 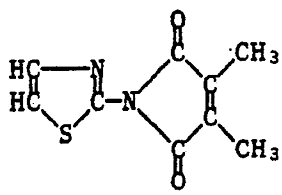 | 10 | 18,7 |
| 6 | 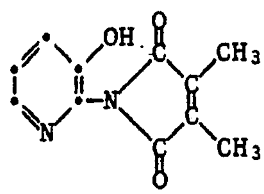 | 10 | 22,8 |
| 7 | 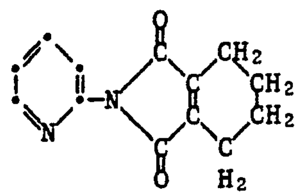 | 10 | 49 |
| 8 | 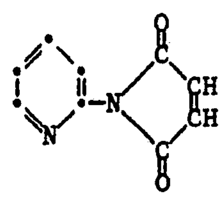 | 10 | 46,7 |
| 9 | 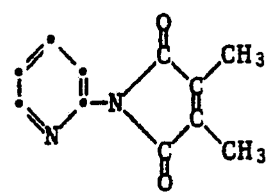 | ~~50 (Versuch 1)~~<br>50 ~~(Versuch 2)~~ | ~~8,7~~<br>15,0 |
| 10 | 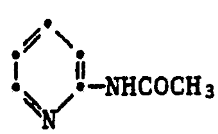 | 10 | 33 |

Tabelle (Fortsetzung)

| Beispiel | Amidin bzw. Amidinsalz | Menge (Mol-%) | Ausbeute DMA (Gewichtsprozent) |
|---|---|---|---|
| 11 | 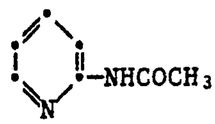 | ~~150 (Versuch 1)~~<br>50 ~~(Versuch 2)~~ | ~~7,1~~<br>30,5 |
| 12 | 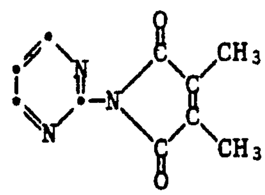 | 10 | 14,6 |
| 13 | 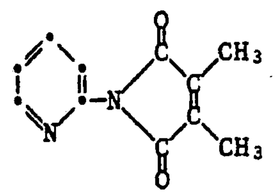 | 10 | 9,8 |
| 14 | 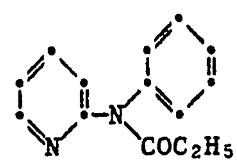 | 10 | 23,8 |

**Patentansprüche**

1. Verfahren zur Herstellung von Dimethylmaleinsäureanhydrid aus 2 Äquivalenten Maleinsäure, Fumarsäure und/oder Maleinsäureanhydrid in Gegenwart eines Amidines, eines Amidinsalzes oder Gemisches davon und bei Temperaturen von mindestens 90°C, dadurch gekennzeichnet, dass das Amidin bzw. das Amidinsalz den Formeln I bzw. II

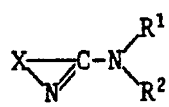 (I)

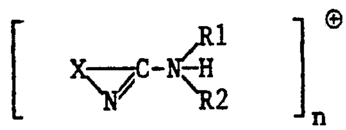 $Y^{n\ominus}$ (II)

entspricht, worin

$R^1$ Acyl und

$R^2$ ein Wasserstoffatom $C_1$-$C_{12}$-Alkyl, $C_5$-$C_7$-Cycloalkyl, $C_6$-$C_{12}$-Aryl, $C_7$-$C_{16}$-Aralkyl, $C_7$-$C_{16}$-Alkaryl, $C_8$-$C_{16}$-Alkaralkyl oder Acyl oder

$R^1$ und $R^2$ zusammen der Diacylrest einer 1,2-Dicarbonsäure sind, Y das Anion einer anorganischen oder organischen Protonensäure und

n eine Zahl von 1 bis 3 bedeuten, und
X zusammen mit der Gruppierung

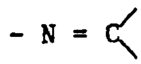

den Rest eines substituierten oder unsubstituierten 5- oder 6-gliedrigen Heteroringes bildet, der weitere Heteroatome enthalten kann.

2. Verfahren gemäss Anspruch 1, dadurch gekennzeichnet, dass die Reaktion in Gegenwart eines inerten Lösungsmittels durchgeführt wird.

3. Verfahren gemäss Anspruch 2, dadurch gekennzeichnet, dass das Lösungsmittel eine aliphatische Carbonsäure mit 2 bis 4 C-Atomen, besonders Essigsäure, ist.

4. Verfahren gemäss Anspruch 1, dadurch gekenneichnet, dass es bei einer Temperatur von 90-200°C durchgeführt wird.

5. Verfahren gemäss Anspruch 1, dadurch gekennzeichnet, dass man bei alleiniger Verwendung von Maleinsäureanhydrid 0,5 bis 20 Gew.-% Wasser einsetzt, bezogen auf die Menge Maleinsäureanhydrid.

6. Verfahren gemäss Anspruch 1, dadurch gekennzeichnet, dass der Heteroring ein Pyridinyl-2-Rest oder ein Thiazolyl-2-Rest ist.

7. Verfahren gemäss Anspruch 1, dadurch gekennzeichnet, dass $R^1$ und $R^2$ als Acyl Acetyl oder Propionyl sind.

8. Verfahren gemäss Anspruch 1, dadurch gekennzeichnet, dass $R^1$ und $R^2$ zusammen als Diacylrest von Phthalsäuren, Maleinsäuren oder Bernsteinsäuren abgeleitet sind.

9. Verfahren gemäss Anspruch 1, dadurch gekennzeichnet, dass Y das Anion einer aliphatischen Monocarbonsäure mit 2 bis 4 C-Atomen, besonders Acetat, ist.

10. Verfahren gemäss Anspruch 1, dadurch gekennzeichnet, dass das Amidin bzw. Amidinsalz der Formel I bzw. II in einer katalytischen Menge von 1 bis 20 Mol-%, bezogen auf die Menge Maleinsäure, Fumarsäure und/oder Maleinsäureanhydrid, eingesetzt wird.

11. Verfahren gemäss Anspruch 1, dadurch gekennzeichnet, dass Maleinsäure, Maleinsäureanhydrid oder 1:1-Gemische hiervon eingesetzt werden.

**Claims**

1. A process for the preparation of dimethylmaleic anhydride from 2 equivalents of maleic acid, fumaric acid and/or maleic anhydride in the presence of an amidine, an amidine salt or a mixture thereof and at temperatures of at least 90°C, in which process the amidine is of formula I and the amidine salt of formula II

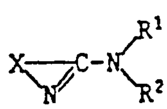 (I)

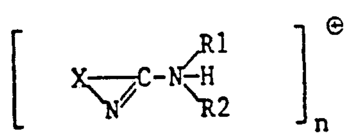 $Y^{n\ominus}$ (II)

in which
$R^1$ is acyl and
$R^2$ is a hydrogen atom, $C_1$-$C_{12}$alkyl, $C_5$-$C_7$cycloalkyl, $C_6$-$C_{12}$aryl, $C_7$-$C_{16}$aralkyl, $C_7$-$C_{16}$alkaryl, $C_8$-$C_{16}$alkaralkyl or acyl or
$R^1$ and $R^2$ together are the diacyl radical of a 1,2-dicarboxylic acid,
Y is the anion of an inorganic or organic protonic acid and
n is an integer from 1 to 3, and X, together with the group

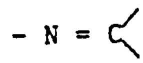

forms the radical of a substituted or unsubstituted 5- or 6-membered heterocyclic ring which may contain further hetero atoms.

2. A process according to claim 1, wherein the reaction is carried out in the presence of an inert solvent.

3. A process according to claim 2, wherein the solvent is an aliphatic carboxylic acid having 2 to 4 carbon atoms, in particular acetic acid.

4. A process according to claim 1, which is carried out at a temperature of from 90 to 200° C.

5. A process according to claim 1, wherein 0.5 to 20 % by weight of water, based on the amount of maleic anhydride, is employed if the maleic anhydride is employed alone.

6. A process according to claim 1, wherein the heterocyclic ring is a 2-pyridyl radical or a 2-thiazolyl radical.

7. A process according to claim 1, wherein $R^1$ and $R^2$ as acyl are acetyl or propionyl.

8. A process according to claim 1, wherein $R^1$ and $R^2$ together as diacyl radicals are derived from phthalic acid, maleic acid or succinic acid.

9. A process according to claim 1, wherein Y is the anion of an aliphatic monocarboxylic acid having 2 to 4 carbon atoms, in particular acetate.

10. A process according to claim 1, wherein the amidine of formula I or the amidine salt of formula II is employed in a catalytic amount of 1 to 20 mol-%, based on the amount of maleic acid, fumaric acid and/or maleic anhydride.

11. A process according to claim 1, which comprises the use of maleic acid, maleic anhydride or a 1 : 1 mixture thereof.

**Revendications**

1. Procédé pour préparer l'anhydride diméthylmaléique à partir de deux équivalents d'acide maléique, d'acide fumarique et/ou d'anhydride maléique en présence d'une amidine, d'un sel d' amidine ou d'un mélange de composés de ce genre et à des températures d'au moins 90°C, procédé caractérisé en ce que l'amidine et le sel d'amidine répondent aux formules I et II :

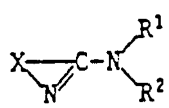

(I)

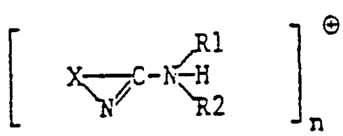

$Y^{n\ominus}$ (II)

dans lesquelles

$R^1$ représente un radical acyle,

$R^2$ représente un atome d'hydrogène, un alkyle en $C_1$-$C_{12}$, un cycloalkyle en $C_5$-$C_7$, un aryle en $C_6$-$C_{12}$, un aralkyle en $C_7$-$C_{16}$, un alkylaryle en $C_7$-$C_{16}$, un alkyl-aralkyle en $C_8$-$C_{16}$ ou un acyle, ou encore

$R^1$ et $R^2$ forment ensemble le radical diacyle d'un acide dicarboxylique-1,2,

Y représente l'anion d'un acide protonique minéral ou organique,

n désigne un nombre de 1 à 3, et

X forme, avec le groupement:

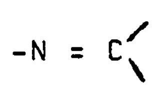 ,

le radical d'un hétérocycle à 5 ou 6 maillons, substitué ou non, qui peut contenir d'autres hétéroatomes.

2. Procédé selon la revendication 1 caractérisé en ce que la réaction est effectuée en présence d'un solvant inerte.

3. Procédé selon la revendication 2 caractérisé en ce que le solvant est un acide carboxylique aliphatique contenant de 2 à 4 atomes de carbone, en particulier l'acide acétique.

4. Procédé selon la revendication 1 caractérisé en ce qu'il est exécuté à une température de 90 à 200° C.

5. Procédé selon la revendication 1 caractérisé en ce que, lorsqu'on se sert de l'anhydride maléique seul, on utilise de 0,5 à 20 % en poids d'eau par rapport à la quantité d'anhydride maléique.

6. Procédé selon la revendication 1 caractérisé en ce que le radical hétérocyclique est un radical pyridyle-2 ou un radical thiazolyle-2.

7. Procédé selon la revendication 1 caractérisé en ce que $R^1$ et $R^2$, en tant que radicaux acyles, sont chacun un radical acétyle ou propionyle.

8. Procédé selon la revendication 1 caractérisé en ce que le radical diacyle que $R^1$ et $R^2$ peuvent former ensemble dérive d'un acide phtalique, d'un acide maléique ou d'un acide succinique.

9. Procédé selon la revendication 1 caractérisé en ce que Y est l'anion d'un acide monocarboxylique aliphatique contenant de 2 à 4 atomes de carbone, en particulier l'anion acétate.

10. Procédé selon la revendication 1 caractérisé en ce que l'amidine ou le sel d'amidine de formule I ou II est mis en jeu en une quantité catalytique, comprise entre 1 et 20 % en moles par rapport à la quantité de l'acide maléique, de l'acide fumarique et/ou de l'anhydride maléique.

11. Procédé selon la revendication 1 caractérisé en ce qu'on utilise l'acide maléique, l'anhydride maléique ou un mélange de ces deux composés dans le rapport 1 : 1.